# EUROPÄISCHE PATENTSCHRIFT

(11) **EP 4 072 658 B1**
(45) Veröffentlichungstag und Bekanntmachung des Hinweises auf die Patenterteilung: **08.11.2023**
(21) Anmeldenummer: 19835606.5
(22) Anmeldetag: 11.12.2019
(51) Int. Cl.: A61N 1/36, A61B 5/00

(54) **VORRICHTUNG ZUR ERTÜCHTIGUNG DES GLEICHGEWICHTSSINNS**
DEVICE FOR STRENGTHENING THE SENSE OF BALANCE
DISPOSITIF POUR RENFORCER LE SENS DE L'ÉQUILIBRE

(43) Veröffentlichungstag der Anmeldung: 19.10.2022
(73) Patentinhaber: Ged Gesellschaft für Elektronik und Design mbH, 53809 Ruppichteroth (DE)
(72) Erfinder: PLATZ, Hanno, 53809 Ruppichteroth (DE)
(74) Vertreter: Angerhausen, Christoph
(86) Internationale Anmeldenummer: PCT/DE2019/101073
(87) Internationale Veröffentlichungsnummer: WO 2021/115505

(56) Entgegenhaltungen:
- EP-A1- 2 592 604
- WO-A1-00/04840
- WO-A1-2017/198337
- DE-A1-102014 222 076
- DE-A1-102015 103 874
- US-A1- 2013 147 611
- US-B1- 6 443 913

## Beschreibung

Die Erfindung betrifft eine Vorrichtung zur Ertüchtigung des Gleichgewichtssinns, die mindestens eine erste und eine zweite akustische Ausgabeeinheit aufweist, die einander zugewandt unter einem Abstand zueinander angeordnet sind. Die Vorrichtung weist weiterhin mindestens einen Neigungssensor auf, der dazu eingerichtet ist, einen Neigungswinkel der Vorrichtung in Bezug auf die Lotrichtung zu messen und an eine Mess- und Steuereinheit auszugeben, wobei die Mess- und Steuereinheit dazu eingerichtet ist, mindestens eine der akustischen Ausgabeeinheiten zur Ausgabe eines akustischen Signals anzusteuern, das abhängig von dem gemessenen Neigungswinkel ist.

Die US 6,443,913 B1 beschreibt eine Vorrichtung zur Linderung von Bewegungskrankheiten und weist einen Bewegungssensor und eine akustische Ausgabeeinheit aufweist. Die Vorrichtung kann als Kopfhörer ausgebildet sein, wobei der Sensor in dem Kopfhörer angeordnet ist und dazu eingerichtet ist, Kopfbewegungen zu erfassen, wobei zur Bestimmung der Kopfbewegung Geschwindigkeiten oder Beschleunigungen des Kopfes erfasst werden, das heißt Positions- oder Winkeländerungen des Kopfes bzw. der Vorrichtung. Je nach vollzogener und erfasster Kopfbewegung können daran angepasste Töne oder Rauschen von Lautsprechern des Kopfhörers ausgegeben werden. Ähnliche Vorrichtungen beschreiben auch die WO 00/004840 A1 und die WO 2013/134873 A1.

Die aus dem Stand der Technik bekannten Vorrichtungen haben den Nachteil, dass die ausgegebenen akustischen Signale vom Anwender häufig nicht intuitiv in eine geeignete Ausgleichsbewegung umgesetzt werden können. Es ist daher die Aufgabe der Erfindung, eine Vorrichtung der eingangs beschriebenen Art derart weiterzuentwickeln, dass sie bereits nach wenigen Übungseinheiten die intuitive Anwendung erlaubt und insbesondere reflektorische Ausgleichsbewegungen eines Anwenders fördert.

Diese Aufgabe wird durch eine Vorrichtung mit den Merkmalen des Anspruchs 1 gelöst. Die abhängigen Ansprüche betreffen jeweils vorteilhafte Ausführungsformen der Erfindung.

Demgemäß ist vorgesehen, dass das akustische Signal eine erste akustisch wahrnehmbare Signalcharakteristik, die unabhängig von dem gemessenen Neigungswinkel ist, und eine zweite akustisch wahrnehmbare Signalcharakteristik aufweist, die in Abhängigkeit von dem gemessenen Neigungswinkel variiert ist. Die erste und die zweite akustisch wahrnehmbare Signalcharakteristik unterscheiden sich voneinander.

Die zweite akustisch wahrnehmbare Signalcharakteristik kann proportional zu dem gemessenen Neigungswinkel variiert sein. Beispielsweise kann die zweite akustisch wahrnehmbare Signalcharakteristik proportional zu dem gemessenen Neigungswinkel vergrößert oder verkleinert sein.

Die erste oder die zweite akustisch wahrnehmbare Signalcharakteristik kann eine akustische Frequenz, eine Signalamplitude, eine Lautstärke oder ein Signalrhythmus sein. Es kann beispielsweise vorgesehen sein, dass abhängig von dem gemessenen Neigungswinkel ein Ton mit einer gleichbleibenden Tonfrequenz, welche die erste, vom Neigungswinkel unabhängige Signalcharakteristik darstellt, abhängig vom Neigungswinkel in seiner Lautstärke, beziehungsweise in seiner Signalamplitude oder Signalintensität variiert wird. Beispielsweise kann die Lautstärke des Tons proportional zum gemessenen Neigungswinkel vergrößert werden, das heißt mit steigender Abweichung der Ausrichtung der Vorrichtung von der Lotrichtung bei gleichbleibender Frequenz lauter werden.

Die Vorrichtung kann mindestens zwei Neigungssensoren und besonders bevorzugt genau zwei Neigungssensoren aufweisen, von denen ein erster Neigungssensor einen Neigungswinkel der Vorrichtung in Bezug auf die Lotrichtung in einer ersten Kippebene misst und von denen ein zweiter Neigungssensor einen Neigungswinkel der Vorrichtung in Bezug auf die Lotrichtung in einer zweiten Kippebene misst, die sich von der ersten Kippebene unterscheidet. Beispielsweise können die beiden Kippebenen der über die Neigungssensoren erfassten Neigungswinkel unter einem Winkel von 90° zueinander angeordnet sein. Die Kippebenen können beispielsweise zwei Symmetrieebenen der Vorrichtung sein, insbesondere zwei vertikale Symmetrieebenen.

Eine der beiden Symmetrieebenen kann beispielsweise parallel zu einer erwarteten Laufrichtung eines Benutzers der Vorrichtung und die andere senkrecht dazu angeordnet sein.

Die Vorrichtung kann insbesondere nach Art eines Kopfhörers ausgebildet sein. Beispielsweise können die erste und die zweite akustische Ausgabeeinheit über einen Kopfhörerbügel miteinander verbunden und unter dem Abstand zueinander angeordnet sein. Dabei kann die Mess- und Steuereinheit an dem Kopfhörerbügel befestigt und über elektrische Leiterbahnen mit den akustischen Ausgabeeinheiten verbunden sein, um diese zur Bereitstellung der akustischen Signale anzusteuern.

Die Mess- und Steuereinheit kann zumindest teilweise oberhalb und/oder mittig zwischen den akustischen Ausgabeeinheiten angeordnet sein, wodurch eine vorteilhafte Gewichtsverteilung der Vorrichtung erreicht wird. Die Mess- und Steuereinheit kann alternativ oder zusätzlich zumindest teilweise in oder an zumindest einer der beiden akustischen Ausgabeeinheiten angeordnet sein. Mess- und Steuereinheit kann beispielsweise in einem Gehäuse seitlich an einem Kopfhörer, welcher die akustischen Ausgabeeinheiten aufweist, angeordnet sein.

Die Mess- und Steuereinheit kann beispielsweise als ein Nachrüstbauteil für gängige Kopfhörer bereitgestellt sein und weist dazu einerseits eine mechanische Schnittstelle für die Befestigung der Mess- und Steuereinheit beziehungsweise eines Gehäuses dieser an dem Kopfhörerbügel sowie eine elektrische Schnittstelle zur Ansteuerung der akustischen Ausgabeeinheiten auf, welche durch die beiden Lautsprechermuscheln eines gängigen Kopfhörers bereitgestellt sein können. Die elektrische Schnittstelle kann elektrische Leiterbahnen aufweisen, von denen eine erste der Leiterbahnen die Mess- und Steuereinheit mit einer ersten der beiden akustischen Ausgabeeinheiten beziehungsweise Kopfhörermuscheln und von denen mit einer zweiten der Leiterbahnen die Mess- und Steuereinheit mit einer zweiten der akustischen Ausgabeeinheiten beziehungsweise Kopfhörermuscheln verbunden ist. Jede der Leiterbahnen kann nur eine einzige oder eine Mehrzahl unabhängiger elektrischer Leiter für die Signalübertragung und/oder elektrische Energieversorgung aufweisen.

Die Mess- und Steuereinheit kann dazu eingerichtet sein, die Ausgabe des akustischen Signals auszusetzen beziehungsweise die akustischen Ausgabeeinheiten dementsprechend anzusteuern, wenn der Neigungswinkel der Vorrichtung in Bezug auf die Lotrichtung Null ist. Alternativ kann vorgesehen sein, dass anstelle des vollständigen Aussetzens des akustischen Signals die Variation der zweiten akustisch wahrnehmbaren Signalcharakteristik unterbrochen wird, so dass sich für einen Benutzer bei einer Ausrichtung der Vorrichtung in der Lotrichtung beziehungsweise in einem Toleranzbereich um die Lotrichtung ein unvariiertes, d.h. monotones Referenzsignal ergibt, welches ihm die korrekte Ausrichtung der Vorrichtung intuitiv signalisiert und aufgrund seiner Monotonität nach kurzer Gewöhnung von einem Anwender kognitiv nicht wahrgenommen wird.

Die Mess- und Steuereinheit kann dazu eingerichtet sein, die Ausgabe des akustischen Signals beziehungsweise die Variation der zweiten akustisch wahrnehmbaren Signalcharakteristik einzusetzen, wenn der gemessene Neigungswinkel einen Mindestwinkel übersteigt. Der Mindestwinkel kann beispielsweise mindestens 5° und besonders bevorzugt mindestens 8° betragen.

Die Mess- und Steuereinheit kann eine Datenschnittstelle, vorzugsweise eine Funkschnittstelle zu einer Recheneinheit aufweisen, wobei die Recheneinheit vorzugsweise als ein Computer oder ein mobiles Endgerät ausgebildet ist, etwa als ein Smartphone oder ein Tablet.

Die Mess- und Steuereinheit kann weiterhin dazu eingerichtet sein, über die Datenschnittstelle die von ihrem Neigungssensor gemessenen Neigungswinkel an die Recheneinheit zu übertragen. Alternativ oder zusätzlich kann die Mess- und Steuereinheit dazu eingerichtet sein, die gemessenen Neigungswinkel unmittelbar nach Ihrer Erfassung durch den Neigungssensor an die Recheneinheit zu übertragen.

Schließlich kann die Mess- und Steuereinheit dazu eingerichtet sein, die gemessenen Neigungswinkel in einem lokalen Speicher der Vorrichtung und vorzugsweise in einem lokalen Speicher der Mess- und Steuereinheit zu speichern.

Die zuvor beschriebene Vorrichtung kann dazu verwendet werden, den Gleichgewichtssinn einer Person zu bewerten und gegebenenfalls zu ertüchtigen. Dabei kann die Information über die Fehllage der Vorrichtung über ein akustisches Signal erfolgen, das aus Sensordaten derart berechnet wird, dass der akustische Höreindruck in einer der Fehllage entsprechenden beziehungsweise physiologisch sinnvoll liegenden Raumrichtung wahrgenommen wird, wodurch die reflektive Auslösung einer Gegenbewegung bei einem Anwender noch weiter verbessert werden kann. So kann beispielsweise vorgesehen sein, dass das akustische Signal und/oder die Variation der zweiten akustisch wahrnehmbaren Signalcharakteristik nur selektiv an einer der beiden akustischen Ausgabeeinheiten ausgegeben wird, um bei einem Benutzer eine intuitive Gegenbewegung auszulösen.

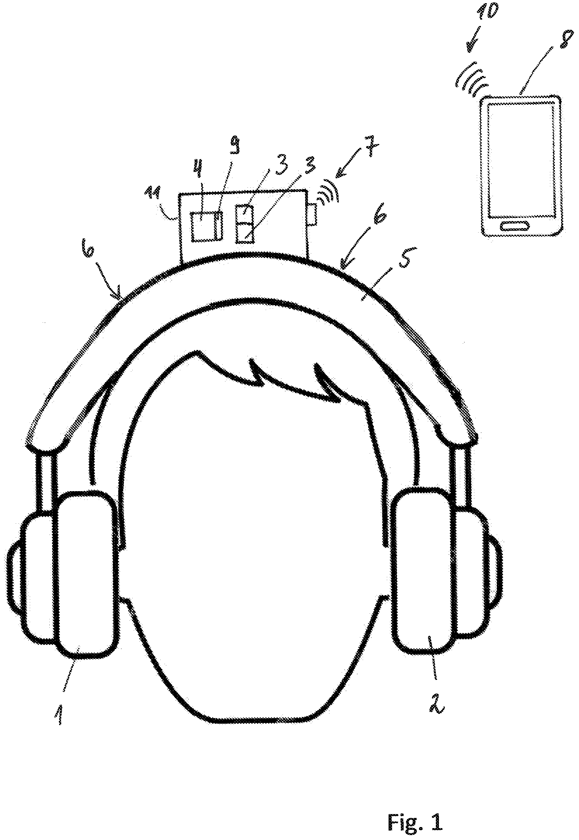

Eine beispielhafte Ausführungsform der Erfindung ist in der nachstehenden Figur 1 gezeigt.

Dabei ist die Vorrichtung nach Art eines Kopfhörers ausgebildet, bei dem die beiden akustischen Ausgabeeinheiten 1, 2 die Lautsprechermuscheln des Kopfhörers bilden. Diese sind über einen Kopfhörerbügel 5 miteinander verbunden, so dass die Vorrichtung analog zu einem Kopfhörer auf dem Kopf getragen werden kann. Mittig außenseitig an dem Kopfhörerbügel 5 ist ein Gehäuse 11 angeordnet, in welchem mindestens ein Neigungssensoren 3 und im vorliegenden Fall zwei Neigungssensoren 3, die Mess- und Steuereinheit 4 und ein Speicher 9 aufgenommen sind. Das Gehäuse 11 weist weiterhin eine Funkschnittstelle 7, beispielsweise eine Bluetooth-Schnittstelle, auf, über die von den Neigungssensoren 3 erfasste und gegebenenfalls von der Mess- und Steuereinheit 4 ausgewertete Signale an ein mobiles Endgerät 8, das beispielsweise ein Smartphone mit einer weiteren Datenschnittstelle 10 sein kann, ausgegeben werden.

Die Neigungssensoren 3 sind dazu vorgesehen, jeweils einen Neigungswinkel der Vorrichtung in Bezug auf die Lotrichtung zu messen und an die Mess- und Steuereinheit 4 auszugeben. Beispielsweise können die beiden Neigungssensoren 3 dazu eingerichtet sein, Neigungswinkel in zwei um 90° zueinander versetzten Ebenen zu bestimmen. Beispielsweise kann eine erste der Ebenen die Zeichnungsebene und eine zweite der Ebene senkrecht zur Zeichnungsebene und in Vertikal- oder Horizontalrichtung ausgerichtet sein. Demgemäß ist bei einer anderen Ausführungsform auch denkbar, dass ein dritter Neigungssensor vorgesehen ist.

Die erfindungsgemäße Vorrichtung kann als Nachrüstlösung für existierende Kopfhörer, beispielsweise für Hifi-Kopfhörer bereitgestellt sein, indem das Gehäuse 11 eine Schnittstelle zu dem Bügel 5 für die mechanische Befestigung des Gehäuses 11 aufweist. Über Leiterbahnen 6, die an der Oberseite des Bügels 5 geführt sein können, kann das Gehäuse 11 beziehungsweise die darin aufgenommene Elektronik mit den beiden akustischen Ausgabeeinheiten 1, 2 für die Signalübertragung verbunden werden.

Die in der vorstehenden Beschreibung, in den Zeichnungen sowie in den Ansprüchen offenbarten Merkmale der Erfindung können sowohl einzeln als auch in beliebiger Kombination für die Verwirklichung der Erfindung wesentlich sein.

### Bezugszeichenliste

- 1: erste akustischer Ausgabeeinheit
- 2: zweite akustische Ausgabeeinheit
- 3: Neigungssensor
- 4: Steuereinheit
- 5: Bügel
- 6: Leiterbahn
- 7: Datenschnittstelle
- 8: Recheneinheit
- 9: Speicher
- 10: weitere Datenschnittstelle
- 11: Gehäuse

## Patentansprüche

1. Vorrichtung zur Ertüchtigung des Gleichgewichtssinns, die mindestens eine erste und eine zweite akustische Ausgabeeinheit (1, 2) aufweist, die einander zugewandt unter einem Abstand zueinander angeordnet sind, wobei die Vorrichtung weiterhin mindestens einen Neigungssensor (3) aufweist, der dazu eingerichtet ist, einen Neigungswinkel der Vorrichtung in Bezug auf die Lotrichtung zu messen und an eine Mess- und Steuereinheit (4) auszugeben, wobei die Mess- und Steuereinheit (4) dazu eingerichtet ist, mindestens eine der akustischen Ausgabeeinheiten (1, 2) zur Ausgabe eines akustischen Signals anzusteuern, das abhängig von dem gemessenen Neigungswinkel ist, **dadurch gekennzeichnet, dass** das akustische Signal eine erste akustisch wahrnehmbare Signalcharakteristik, die unabhängig von dem gemessenen Neigungswinkel ist, und eine zweite akustisch wahrnehmbare Signalcharakteristik aufweist, die in Abhängigkeit von dem gemessenen Neigungswinkel variiert ist, wobei die Mess- und Steuereinheit (4) weiterhin dazu eingerichtet ist, die Ausgabe des akustischen Signals auszusetzen, wenn der Neigungswinkel der Vorrichtung in akustischen Signals auszusetzen, wenn der Neigungswinkel der Vorrichtung in Bezug auf die Lotrichtung Null ist, wobei die Mess- und Steuereinheit (4) dazu einerichtet ist, die Ausgabe des aktustischen Signals einzusetzen, wenn der gemessene Neigungswinkel einen Mindestwinkel übersteigt, wobei der Mindestwinkel vorzugsweise mindestens 5 ° und besonders bevorzugt mindestens 8 ° beträgt.

2. Vorrichtung nach Anspruch 1, bei der die zweite akustisch wahrnehmbare Signalcharakteristik proportional zu dem gemessenen Neigungswinkel variiert ist.

3. Vorrichtung nach Anspruch 2, bei der die zweite akustisch wahrnehmbare Signalcharakteristik proportional zu dem gemessenen Neigungswinkel vergrößert oder verkleinert ist.

4. Vorrichtung nach einem der vorangegangenen Ansprüche, bei der die erste oder die zweite akustisch wahrnehmbare Signalcharakteristik eine akustische Frequenz, eine Signalamplitude, eine Lautstärke oder ein Signalrhythmus ist.

5. Vorrichtung nach einem der vorangegangenen Ansprüche, die mindestens zwei Neigungssensoren (3) aufweist, von denen ein erster Neigungssensor (3) einen Neigungswinkel der Vorrichtung in Bezug auf die Lotrichtung in einer ersten Kippebene misst und von denen ein zweiter Neigungssensor (3) einen Neigungswinkel der Vorrichtung in Bezug auf die Lotrichtung in einer zweiten Kippebene misst, die sich von der ersten Kippebene unterscheidet.

6. Vorrichtung nach Anspruch 5, die zwei Neigungssensoren (3) aufweist, wobei die beiden Kippebenen der über die Neigungssensoren (3) erfassten Neigungswinkel unter einem Winkel von 90° zueinander angeordnet sind.

7. Vorrichtung nach einem der vorangegangenen Ansprüche, bei der die erste und die zweite akustische Ausgabeeinheit (1, 2) über einen Kopfhörerbügel (5) miteinander verbunden und unter dem Abstand zueinander angeordnet sind.

8. Vorrichtung nach Anspruch 7, bei der die Mess- und Steuereinheit (4) an dem Kopfhörerbügel (5) befestigt und über elektrische Leiterbahnen (6) mit den akustischen Ausgabeeinheiten (1, 2) verbunden ist.

9. Vorrichtung nach Anspruch 7 oder 8, bei der die Mess- und Steuereinheit (4) oberhalb von und mittig zwischen den akustischen Ausgabeeinheiten (1, 2) angeordnet ist.

10. Vorrichtung nach Anspruch 7 oder 8, bei der die Mess- und Steuereinheit (4) zumindest teilweise in zumindest einer der beiden akustischen Ausgabeeinheiten (1, 2) angeordnet ist.

11. Vorrichtung nach einem der vorangegangenen Ansprüche, bei der die Mess- und Steuereinheit (4) eine Datenschnittstelle (7), vorzugsweise eine Funkschnittstelle zu einer Recheneinheit (8) aufweist, vorzugsweise zu einem Computer oder zu einem mobilen Endgerät, etwa einem Smartphone oder einem Tablet.

12. Vorrichtung nach Anspruch 11, bei der die Mess- und Steuereinheit (4) dazu eingerichtet ist, über die Datenschnittstelle (7) die von dem Neigungssensor (3) gemessenen Neigungswinkel an die Recheneinheit (8) zu übertragen.

13. Vorrichtung nach Anspruch 12, bei die Mess- und Steuereinheit (4) dazu eingerichtet ist, die gemessenen Neigungswinkel unmittelbar nach ihrer Erfassung durch den Neigungssensor (3) an die Recheneinheit (8) zu übertragen.

14. Vorrichtung nach einem der vorangegangenen Ansprüche, bei der die Mess- und Steuereinheit (4) dazu eingerichtet ist, die gemessenen Neigungswinkel in einem lokalen Speicher (9) der Vorrichtung und vorzugsweise der Mess- und Steuereinheit (4) zu speichern.

## Claims

1. Device for training the sense of equilibrium which comprises at least a first and a second acoustic output unit (1, 2) facing each other at a distance from each other, the device further comprising at least one inclination sensor (3) arranged to measure an angle of inclination of the device with respect to the vertical direction and to output it to a measuring and control unit (4), the measuring and control unit (4) being arranged to trigger at least one of the acoustic output units (1, 2) to output an acoustic signal, which is dependent on the measured angle of inclination, **characterized in in that** the acoustic signal has a first acoustically perceptible signal characteristic, which is independent of the measured angle of inclination, and a second acoustically perceptible signal characteristic, which varies according to the measured angle of inclination, the measurement and control unit (4) being further arranged to suspend the output of the acoustic signal, when the angle of inclination of the device with respect to the vertical direction is zero, wherein the measuring and control unit (4) is arranged to trigger the output of the acoustic signal when the measured angle of inclination exceeds a minimum angle, wherein the minimum angle is preferably at least 5 ° and particularly preferably at least 8 °.

2. The device of claim 1, wherein the second acoustically perceptible signal characteristic is varied in proportion to the measured angle of inclination.

3. The device of claim 2, wherein the second acoustically perceptible signal characteristic is increased or decreased in proportion to the measured angle of inclination.

4. The device of any one of the preceding claims, wherein the first or second acoustically perceptible signal characteristic is an acoustic frequency, a signal amplitude, a volume, or a signal rhythm.

5. A device according to any one of the preceding claims, comprising at least two inclination sensors (3), of which a first inclination sensor (3) measures an angle of inclination of the device with respect to the vertical direction in a first tilting plane and of which a second inclination sensor (3) measures an angle of inclination of the device with respect to the vertical direction in a second tilting plane which differs from the first tilting plane.

6. The device according to claim 5, comprising two inclination sensors (3), wherein the two tilting planes of the inclination angles detected via the inclination sensors (3) are arranged at an angle of 90° to each other.

7. Device according to any one of the preceding claims, wherein the first and second acoustic output units (1, 2) are connected to each other via a headphone bracket (5) and are spaced apart from each other.

8. The device according to claim 7, wherein the measurement and control unit (4) is attached to the headphone bracket (5) and connected to the acoustic output units (1, 2) via electrical conductors (6).

9. Device according to claim 7 or 8, wherein the measuring and control unit (4) is arranged above and centrally between the acoustic output units (1, 2).

10. The device according to claim 7 or 8, wherein the measurement and control unit (4) is at least partially arranged in at least one of the two acoustic output units (1, 2).

11. Device according to any one of the preceding claims, in which the measurement and control unit (4) has a data interface (7), preferably a radio interface to a computing unit (8), preferably to a computer or to a mobile device, such as a smartphone or a tablet.

12. The device according to claim 11, wherein the measuring and control unit (4) is arranged to transmit the inclination angles measured by the inclination sensor (3) to the computing unit (8) via the data interface (7).

13. Device according to claim 12, wherein the measuring and control unit (4) is arranged to transmit the measured inclination angles to the computing unit (8) immediately after their detection by the inclination sensor (3).

14. Device according to any one of the preceding claims, wherein the measuring and control unit (4) is arranged to store the measured inclination angles in a local memory (9) of the device and preferably of the measuring and control unit (4).

## Revendications

1. Dispositif pour le renforcement du sens de l'équilibre, qui comprend au moins une première et une deuxième unités d'émission acoustique (1, 2), qui sont disposées de manière orientée l'une vers l'autre avec une certaine distance entre elles, dans lequel le dispositif comprend en outre au moins un capteur d'inclinaison (3) qui est conçu pour mesure un angle d'inclinaison du dispositif par rapport à la verticale et pour l'envoyer à une unité de mesure et de commande (4), dans lequel l'unité de mesure et de commande (4) est conçue pour contrôler au moins une des unités d'émission acoustique (1, 2) pour l'émission d'un signal acoustique qui dépend de l'angle d'inclinaison mesuré, **caractérisé en ce que** le signal acoustique présente une première caractéristique de signal perceptible acoustiquement, qui est indépendante de l'angle d'inclinaison mesuré, et une deuxième caractéristique de signal perceptible acoustiquement qui varie en fonction de l'angle d'inclinaison mesuré, dans lequel l'unité de mesure et de commande (4) est en outre conçue pour suspendre l'émission du signal acoustique lorsque l'angle d'inclinaison du dispositif par rapport à la verticale est égal à zéro, dans lequel l'unité de mesure et de commande (4) est conçue pour activer l'émission du signal acoustique lorsque l'angle d'inclinaison mesuré dépasse un angle minimal, dans lequel l'angle minimal est de préférence d'au moins 5° et plus particulièrement de préférence d'au moins 8°.

2. Dispositif selon la revendication 1, dans lequel la deuxième caractéristique de signal perceptible acoustiquement varie proportionnellement à l'angle d'inclinaison mesuré.

3. Dispositif selon la revendication 2, dans lequel la deuxième caractéristique de signal perceptible acoustiquement est augmentée ou diminuée proportionnellement à l'angle d'inclinaison mesuré.

4. Dispositif selon l'une des revendications précédentes, dans lequel la première ou la deuxième caractéristique de signal perceptible acoustiquement est une fréquence acoustique, une amplitude de signal, un volume sonore ou un rythme du signal.

5. Dispositif selon l'une des revendications précédentes, qui comprend au moins deux capteurs d'inclinaison (3) dont un premier capteur d'inclinaison (3) mesure un angle d'inclinaison du dispositif par rapport à la verticale dans un premier plan de basculement et dont un deuxième capteur d'inclinaison (3) mesure un angle d'inclinaison du dispositif par rapport à la verticale dans un deuxième plan de basculement, qui est différent du premier plan de basculement.

6. Dispositif selon la revendication 5, qui comprend deux capteurs d'inclinaison (3), dans lequel les deux plans de basculement des angles d'inclinaison mesurés par les capteurs d'inclinaison (3) sont disposés entre eux avec un angle de 90°.

7. Dispositif selon l'une des revendications précédentes, dans lequel les première et deuxième unité d'émission acoustique (1, 2) sont reliées entre elles par l'intermédiaire d'un étrier de casque audio (5) et sont disposées avec une distance entre elles.

8. Dispositif selon la revendication 7, dans lequel l'unité de mesure et de commande (4) est fixée à l'étrier de casque audio (5) et est reliée, par l'intermédiaire de pistes conductrices électriques (6), avec les unités d'émission acoustique (1, 2).

9. Dispositif selon la revendication 7 ou 8, dans lequel l'unité de mesure et de commande (4) est disposée au-dessus et au centre entre les unités d'émission acoustique (1, 2).

10. Dispositif selon la revendication 7 ou 8, dans lequel l'unité de mesure et de commande (4) est disposée au moins partiellement dans au moins une des deux unités d'émission acoustique (1, 2).

11. Dispositif selon l'une des revendications précédentes, dans lequel l'unité de mesure et de commande (4) comprend une interface de données (7), de préférence une interface radio, avec une unité de calcul (8), de préférence un ordinateur ou un terminal mobile, par exemple un smartphone ou une tablette.

12. Dispositif selon la revendication 11, dans lequel l'unité de mesure et de commande (4) est conçue pour transmettre, par l'intermédiaire de l'interface de données (7), les angles d'inclinaison mesurés par le capteur d'inclinaison (3) à l'unité de calcul (8).

13. Dispositif selon la revendication 12, dans lequel l'unité de mesure et de commande (4) est conçue pour transmettre les angles d'inclinaison mesurés directement après leur mesure par le capteur d'inclinaison (3) à l'unité de calcul (8).

14. Dispositif selon l'une des revendications précédentes, dans lequel l'unité de mesure et de commande (4) est conçue pour enregistrer les angles d'inclinaison mesurés dans une mémoire locale (9) du dispositif et de préférence de l'unité de mesure et de commande (4).
